# EUROPEAN PATENT SPECIFICATION

(11) **EP 3 883 464 B1**
(45) Date of publication and mention of the grant of the patent: **23.04.2025**
(21) Application number: 20706012.0
(22) Date of filing: 08.01.2020
(51) Int. Cl.: A61B 5/06, A61B 5/00, A61F 2/95

(54) **INTEGRATED IMAGING AND MEDICAL DEVICE DELIVERY APPARATUS**
INTEGRIERTE BILDGEBUNGS- UND MEDIZINPRODUKTFREISETZUNGSVORRICHTUNG
APPAREIL D'IMAGERIE INTÉGRÉ ET DE POSE DE DISPOSITIF MÉDICAL

(30) Priority: 08.01.2019 GB 201900264; 06.08.2019 GB 201911259
(43) Date of publication of application: 29.09.2021
(73) Proprietor: Vascutek Limited, Renfrewshire PA4 9RR (GB)
(72) Inventor: RUMBLES, Robert, Crosslee Renfrewshire PA6 7BW (GB); CHOWDHURY, Aliah, Glasgow G12 8JR (GB)
(74) Representative: Murgitroyd & Company
(86) International application number: PCT/GB2020/050036
(87) International publication number: WO 2020/144477

(56) References cited:
- WO-A1-2006/076328
- US-A1- 2002 077 643
- US-A1- 2009 234 445
- US-A1- 2010 094 259
- US-A1- 2011 301 685
- US-A1- 2014 180 126
- US-A1- 2015 105 818

## Description

The present invention relates to an apparatus and method for use in imaging-guided vascular surgery and particularly to such an apparatus and method for delivering medical devices such as stents.

Imaging-guided vascular surgery is widely practiced, typically under x-ray, but increasingly employs an intravascular imaging probe mounted on a standalone guided catheter to inspect the vascular site prior to or after treatment. In this connection, intravascular imaging modalities known to be particularly effective for aiding treatment of vascular diseases are Intravascular Ultrasound (IVUS) and Optical Coherence Tomography (OCT), with the pros and cons of each known to be complementary to one another. Advantages of IVUS include the elimination of the need to use contrast and the feature that IVUS has greater depth of field. However, IVUS can suffer from clarity issues.

In comparison, OCT requires contrast and has less depth of sensory penetration, but produces superior image resolution and thus yields better edge detection. Therefore, it is widely acknowledged in the technical field that it is advantageous to combine multiple imaging modalities onto a single imaging probe to benefit from complementary functions; for example, by mounting an ultrasound transducer and an optical transducer on a single head of such a probe.

Standalone IVUS imaging probes are particularly known to be used in conjunction with x-ray imaging during standard endovascular procedures such as treating aortic aneurysms, to eliminate the need to use contrast to detect branch vessels, which is a benefit to patients who are incompatible with contrast agents. While using x-ray is a standard practice during such procedures to roadmap vessels and detect radiopaque markers on medical devices, other imaging modalities are known to have the capacity to do the same with the appropriate image registration capability, which enables them to be used interchangeably with, or entirely replacing x-ray, thereby reducing or removing the patient's exposure to radiation.

Procedurally, the use of independent guided imaging catheters to deliver a medical device to a treatment site adds invasive procedural steps by inserting and withdrawing the independent imaging catheter as well as the required medical device delivery apparatus.

In this respect, the method of delivering and deploying a medical device such as a stent graft using an independent imaging catheter typically takes the following steps:-
1. Insert the guided imaging catheter on a guidewire to the treatment site;
2. Roadmap the target vessel either by switching on x-ray to map the relative position of the imaging probe as it detects features of the vessel environment; or pull the probe back through the vessel to reconstruct a linear representation of the vessel by a series of cross sectional images, before advancing the probe back to the treatment site;
3. Insert the stent delivery apparatus on a different guidewire and optionally use x-ray to validate position of stent relative to imaging probe;
4. Deploy the medical device at the treatment site;
5. As the probe will be external the deployed medical device, it will need to be withdrawn before being re-advanced through the lumen of the medical device to be able to inspect the landing zone of the deployed device.

The above procedure is somewhat generalised and may be adapted to suit different anatomical treatments and medical devices; for example, bifurcate body devices need to be positioned just below the renal branches and above the aortic bifurcation, and leg devices need to be positioned just above the iliac branches.

Nonetheless, the key steps of 2 and 3 require road-mapping and measuring the vessel environment. This necessitates either x-ray exposure or an intravascular imaging probe being present in the vessel but exterior to the device delivery apparatus which can be traumatic. Step 5 illustrates the additional steps to withdraw and re-advance the imaging catheter independently of the device delivery apparatus, making it more invasive than a standard procedure such as for EVAR (EndoVascular Aneurysm Repair).

It has therefore now become a known concept to integrate imaging apparatus on a treatment catheter, usually by fixing a stationary imaging probe at a distal or proximal position relative to a device such as a stent graft, valve or fixture, significantly reducing the procedure time and number of steps. It also reduces the traumatic effect of repeated insertion and withdrawal of the imaging catheter for vessel measurement and post treatment inspection separately from the insertion and withdrawal of the stent graft delivery apparatus.

US2009/234445A1 discloses an integrated imaging and medical device delivery apparatus with an imaging sensor and with a position sensor.

In this regard, International patent application WO2004051579 teaches that vessel road-mapping can also be achieved by an IVUS probe that may be coupled on the same catheter as the stent to be deployed. In such a case, the combined guided catheter would advance to the site, pull back through the vessel to roadmap the vessel and its branches, and once again advance to the appropriate position for stent deployment. The imaging probe can inspect stent apposition without having to withdraw the delivery apparatus, being on the same catheter.

US2009/0234445 discloses a catheter device comprising a flexible catheter sheath enclosing a catheter cavity; and at least one sensor for imaging.

WO2006/076328 discloses a delivering system for deploying a stent comprising a catheter with a lumen; and at least one string for releasable attachment to the stent.

US2011301685 discloses a bidirectional stent delivery system including an inner elongated shaft, an expandable prosthesis, an outer elongate shaft and sheath over the prosthesis.

US2002/0077643 discloses a method of introducing an ultrasonic probe into a blood vessel to destroy, reduce or remove tissue with an ultrasonic device.

While there is some disclosure of methods of using an imaging probe at a specific position of the same catheter as a stent or similar medical device, either as a fixed imaging element or integrating a separate imaging probe, there are key procedural and practical limitations of integrating a probe in a fixed location on the delivery apparatus relative to the stent. These limitations include:
1. In practice, many medical treatments require multiple medical devices to be deployed so fixing an imaging probe on each delivery apparatus can be costly to manufacture, as well as the risk of any of the imaging probes failing.
2. Fixing a probe at either end of the stent limits the practical capability to check that the opposite, non-visible end of the stent has cleared any adjacent branch vessel to prevent obstruction. This can only be achieved by necessitating the use of x-ray as part of the procedure and repeatedly manoeuvring the combined catheter longitudinally to align the fixed imaging probe with the features of the vessel.
3. Prior art disclosures of intra-lumenal integration of a separate imaging catheter into various surgical apparatus, including treatment catheters, do not disclose how such an assembly can be detailed to resolve unmet procedural issues in delivering and inspecting deployment of devices without occluding branch vessels at either end.

There are also practical issues arising from the need to maintain a low profile of delivery apparatus containing compacted endovascular devices.

The present invention as such seeks to alleviate the problems identified above.

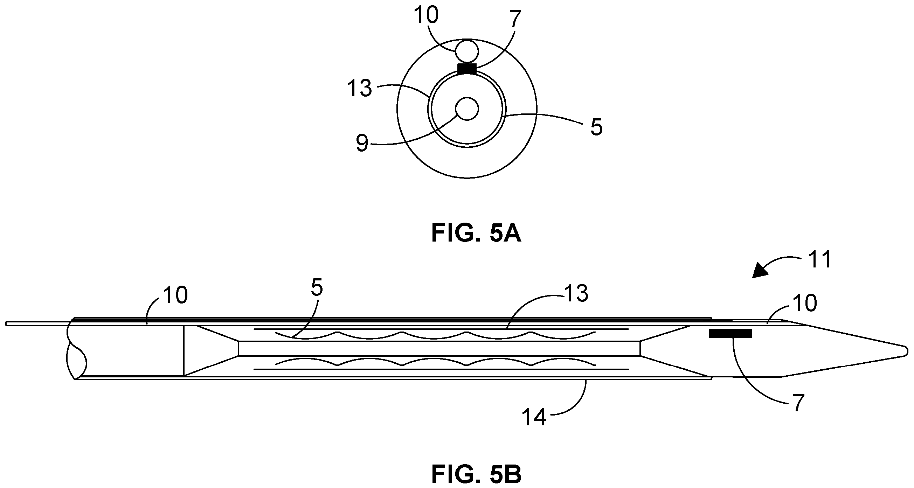

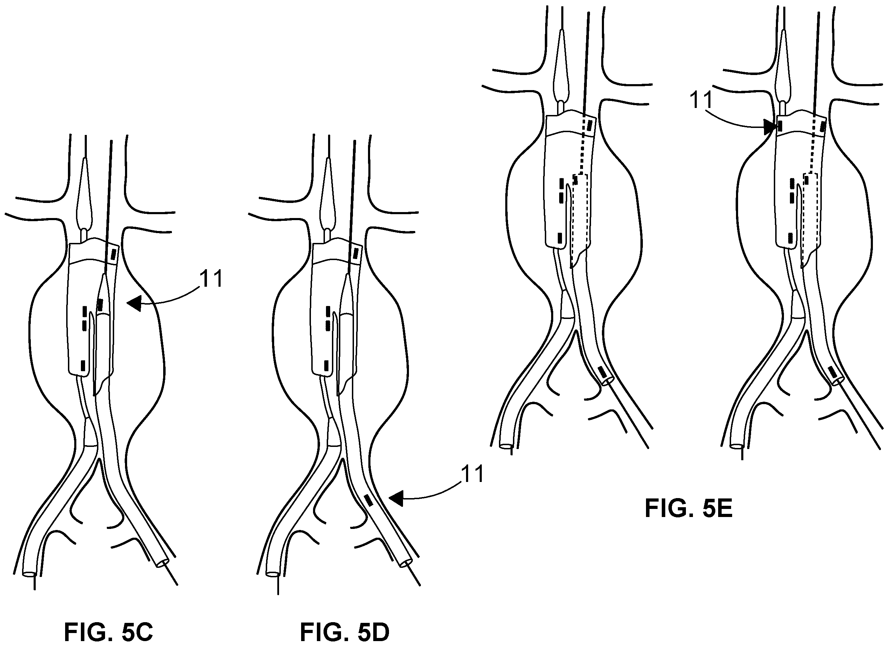

According to the present invention there is provided integrated imaging and medical device delivery apparatus as claimed in the claims. The invention is illustrated by a nonlimiting example in Figure 5.

Preferably, the one or more reference points comprise markers positioned along the length of the navigation pathway. When the imaging probe is positioned at such a reference point, and it has identified certain local site features, such as branch vessels, the relative positioning of a medical device carried by the apparatus can be readily ascertained, since it is at a known distance from the marker. In this way, the position of medical device can be adjusted as required for deployment.

Conveniently, the one or more reference points comprise a restriction in the imaging probe lumen along the navigation pathway, dimensioned to impede further progression of an imaging probe along the imaging probe lumen. Again, with the imaging probe at a known proximal position, due to the restriction, its relative distance from the medical device is known so that the apparatus can be manoeuvred as required for landing of the medical device.

Preferably, in use a deployable medical device is positioned towards a proximal end of the apparatus with its proximal end distanced from the proximal end of the apparatus by a predetermined amount.

In preferred embodiments the apparatus further comprises locking means for locking the imaging probe in position along the navigation pathway.

According to an example not covered by the invention, the imaging probe lumen extends internally of a deployable medical device being retained. In this way, the imaging probe can be advanced and retracted internally of the apparatus, thus minimising trauma resulting from such movement. The internalisation of the imaging probe lumen moreover means the profile size of the delivery apparatus does not need to be altered or increased.

According to an example not covered by the invention, an imaging probe lumen extends through a central shaft of the apparatus. Conveniently, at least part of the central shaft is formed of spectrally transparent material, thereby enhancing the information available.

According to an example not covered by the invention, the imaging probe lumen is externalised from the central shaft but extends internally of a deployable medical device being retained.

According to the invention, the imaging probe lumen extends within the apparatus and externally of a deployable medical device being retained. In this manner, the imaging probe can be used to image the external environment above and below the medical device before its deployment. Once the medical device has been unsheathed, the imaging probe can be advanced internally within the expanded medical device to inspect the site without damage to, for example, the vessel wall. In this respect, in certain embodiments, the imaging probe lumen provided externally of a deployable medical device is a first one of multiple probe lumens, a second imaging probe lumen passing centrally through the apparatus for allowing an imaging probe to be introduced internally within the deployable medical device.

The apparatus further comprises inner and outer sheaths, the inner sheath being for retaining the medical device against deployment and the outer sheath encapsulating the imaging probe lumen provided between the inner sheath and the exterior of the apparatus. As such, during the medical device delivery process prior to unsheathing, the outer sheath provides an outer cover or shield against contamination, and facilitates the movement of the apparatus within the local environment.

Conveniently, the deployable medical device for use with the apparatus comprises a substantially elongate tubular member. Preferably, the deployable medical device comprises a stent device. Such stent devices may be of the expanding variety that are retained in a compacted state by a sheath prior to deployment.

Conveniently, the apparatus further comprises an atraumatic tip provided at the proximal end of the apparatus. Preferably, the or each imaging probe lumen extends into said tip.

Conveniently, the restriction in the imaging probe lumen impedes movement of an imaging probe along said tip past a predefined point. In preferred embodiments, the restriction is provided by way of a reduced diameter section of the lumen, which remains open ended to allow air to be flushed from the lumen.

Preferably, the navigation pathway comprises a visualization section, the reference points being provided within the visualization section for enabling measurement and orientation awareness for the imaging probe. The apparatus as such provides measurable manoeuvrability of the imaging probe to asses positioning at either end of the medical device relative to the site.

A method, not according to the invention, wherein the method of deploying a medical device using an apparatus defined above -, the method comprising the steps:-inserting an imaging probe at a distal-most extent of said navigation pathway; advancing the imaging probe to a proximal extent of the apparatus for one or more of imaging, road-mapping or measuring a site; and moving the imaging probe relative to the site by either stabilising the position of the imaging probe on the apparatus and moving the entire apparatus, or stabilising the position of the apparatus relative to the site and moving the imaging probe within the apparatus.

In this regard, by integrating imaging and deploying features into a method of use of a single tool, the deployment of a medical device such as a stent can be enhanced.

Preferably, the imaging probe is moved relative to the site by stabilising the position of the imaging probe on the apparatus at a desired position relative to the site and moving the entire apparatus until the imaging probe visualises detectable features prior to deployment of the medical device.

Conveniently, the imaging probe is moved relative to the site by stabilising the position of the apparatus relative to the site and moving the imaging probe within the apparatus to visualise the landing zone of the deployed medical device. As such, the deployed device can be inspected and repositioned if necessary.

Preferably, the method further comprises moving the imaging probe to the proximal extent defined by said one or more reference points; locating desired site features using the probe and adjusting the position of the apparatus if required so that the proximal end of the medical device is at a suitable deployment position

A method, not according to the invention, of deploying a medical device using an apparatus as defined above where the imaging probe lumen extends within the apparatus and externally of a deployable medical device being retained, the method comprising the steps:- inserting an imaging probe at a distal-most extent of said navigation pathway of said imaging lumen; advancing the imaging probe to a proximal extent of the apparatus for one or more of imaging, road-mapping or measuring a site; and moving the imaging probe relative to the site to identify an appropriate landing site for the medical device by either stabilising the position of the imaging probe relative to the apparatus and moving the entire apparatus, or stabilising the position of the apparatus relative to the site and moving the imaging probe relative to the apparatus; once an appropriate landing site for the deployable medical device is determined and prior to medical device deployment, withdrawing the imaging probe distally along the imaging lumen, until the imaging probe detects a distal marker on the deployable medical device and confirms its position relative to the site; and deploying the device.

Preferably, the method further comprises inserting an imaging probe internally of said deployed medical device and advancing internally through the deployed medical device for inspection of the site. A second imaging lumen provided internally of the deployed medical device may be provided for this purpose.

In this way, the method can be used for imaging of the exterior environment above and below the device before deployment and being able to advance internally through the expanded device to inspect the site without interference.

According to a further aspect of the present invention there is provided a medical device delivery system, comprising:- an integrated imaging and medical device delivery apparatus as defined above and as claimed; an expandable sheathed medical device retained by said retaining means; and an imaging probe compatible with said imaging probe lumen.

Certain embodiments of the invention will now be described by way of example and with reference to the accompanying drawings:- of which
Figure 1 shows a general figure of an apparatus according to a configuration;
Figures 2a, 2b, 2c, 2d and 2e show another configuration;
Figures 3a, 3b, and 3c, show a second configuration;
Figures 4a, and 4b, show a third configuration; and
Figures 5a to 5e show an embodiment of the present invention.

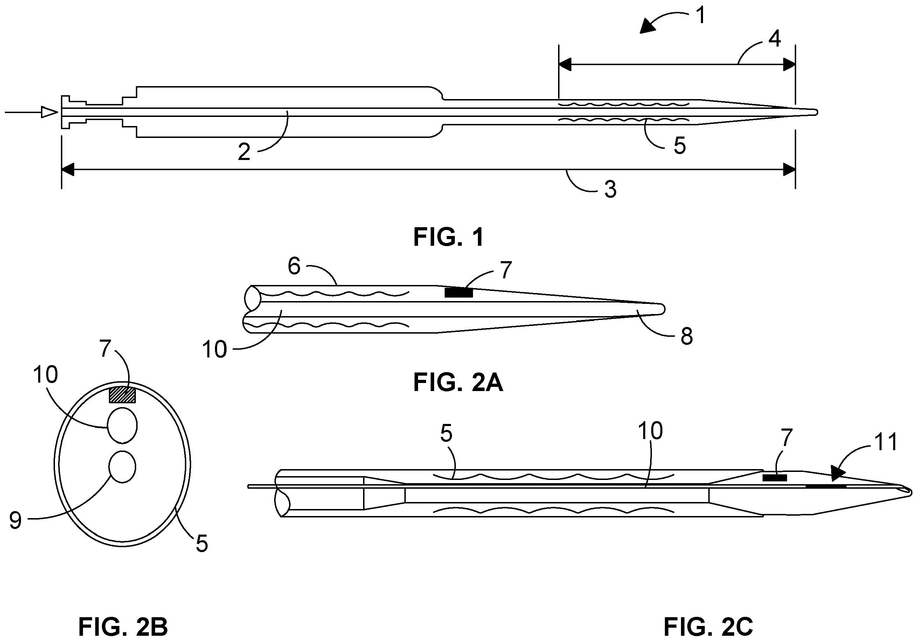

Figure 1 shows a general view of elements of a medical device delivery system 1, incorporating an integrated imaging and medical device delivery apparatus. In this regard, the apparatus comprises a central shaft 2 in which an imaging probe lumen is formed, and through which an imaging probe can travel along a navigation pathway 3. A visualisation section 4 at a proximal end of the apparatus extends from the distal end of a self-expanding stent graft 5 provided within the apparatus, to a proximal end of the apparatus.

More detailed views of a first configuration are shown in Figures 2a to 2c. In this regard, as shown a compacted endovascular device 5 is constrained by outer sheath 6 in the proximal area of the apparatus. A marker 7 is provided in a tip portion 8 at the proximal end of the apparatus.

Guide wire and imaging probe lumens 9, 10 extend through the shaft 2 from the distal end to the proximal end of the apparatus, the lumens running longitudinally and internally through the compacted stent graft 5.

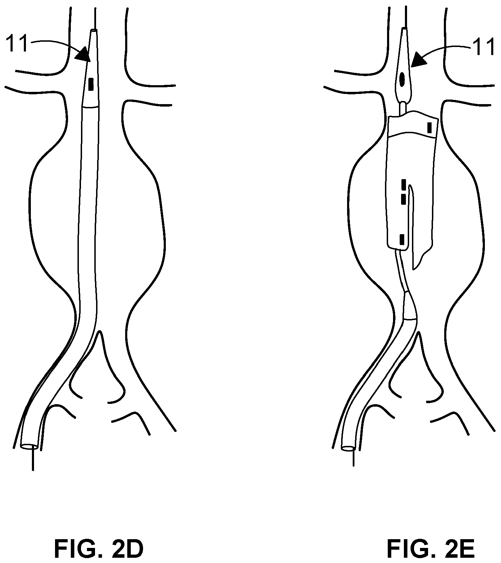

Referring to Figures 2d and 2e, in the method of operation of the apparatus of the first configuration, the apparatus is inserted into a target vessel. For pullback imaging probes to image and reconstruct the vessel, a probe 11 can be advanced beyond the tip to the treatment site and then pulled back the probe until it is within the delivery apparatus. Alternatively, the delivery apparatus catheter can be advanced with the probe 11 integrated at the proximal limit, and then be pulled back, and then re-advanced back to the site. Integrating forward-facing imaging probes can image while advancing the whole delivery apparatus through the vessel to the site.

When the integrated probe 11 is at a proximal limit identified by one or more reference points such as marker 7, it is at a defined distance from the on-board medical device 5. The probe thus can locate the branch vessels and the delivery apparatus can be adjusted if needed so that the proximal end of the device is at a relative known distance clear of the branch vessels above, and at a suitable deployment position. As such, this can be achieved at the same time as road-mapping.

Once in position the medical device 5 can be unsheathed. To check or adjust apposition, the imaging probe can be withdrawn along its visualisation section. The landing zone for the medical device can be inspected from within the expanded device, so that it can be repositioned as needed.

Using such apparatus, a method of use is particularly pertinent in multi-device deployment procedures, such as the standard procedure to treat an Abdominal Aortic Aneurysm (AAA).

The present invention hence relates to an integrated imaging and medical device delivery apparatus, preferably comprising one or more of:-
a) a self-expanding stent graft medical device
   - with radiopaque markers;
   - compacted around a central shaft; and
   - constrained by an introducer sheath.
b) A central shaft
   - with a designated lumen defining the navigation pathway for the imaging probe to be integrated into the apparatus;
   - with an open proximal end at the tip of the shaft; and
   - of a spectrally transparent material.
c) One or more fixed features (spectrally detectable by chosen imaging modalities)
   - such as markers;
   - at known distances along the visualization section relative to the ends of the medical device to enable measurement and orientation awareness for the imaging probe; and
   - at the tip defining the proximal limit of the visualization section at a known distance from the proximal end of the medical device.
d) An imaging probe on an independent catheter
   - of a size compatible with the designated shaft lumen; and
   - controlled by standard components such as Tuohy Borst valves to be advanced, withdrawn or fixed in position to prevent unwanted displacement of the imaging probe.

The present invention thus relates to an endovascular imaging and medical device delivery apparatus and system, with provisions to integrate and remove a compatible independent intravascular imaging probe, preferably comprising one or more of :
A. An onboard medical device with radiopaque markers.
B. An integral navigation pathway defined as the length from the distal-most extent of the delivery apparatus at which the imaging probe can be inserted or removed, to the proximal-most extent that the imaging probe can be navigated to within the delivery apparatus.
C. A visualisation section defined as the portion of the navigation pathway that is conceptually end-limited by features detectable by the imaging probe at a known distance from respective ends of the onboard medical device.
D. A window of spectral visibility of the environment external to the delivery apparatus.

A method of using a medical device delivery apparatus and system with provisions to integrate and remove a compatible independent intravascular imaging probe, having one or more of:
1. Insertion or removal of an independent imaging probe at the distal-most extent of (B).
2. Freedom of movement in either direction for the imaging probe to be advanced or withdrawn along the length of (B).
3. Advancing the imaging probe to the proximal-most extent of (D) to image, roadmap and measure the vessel.
4. Free displacement of the imaging probe along (C) relative to the medical device delivery apparatus by moving one longitudinally and stabilising the other stationary.

a. Displacing the imaging probe along (C) by stabilising the imaging probe stationary at a desired position relative to the vessel and moving delivery apparatus along the vessel until the probe visualises detectable features such as radiopaque markers to desirably position the medical device relative to the road-mapped vessel environment such as branches, prior to deploying the medical device.
b. Displacing the imaging probe along (C) by stabilising the delivery apparatus stationary relative to the vessel and moving the imaging probe within the delivery apparatus to visualise the landing zone of the expanded medical device to enable inspection or repositioning of the device if needed.

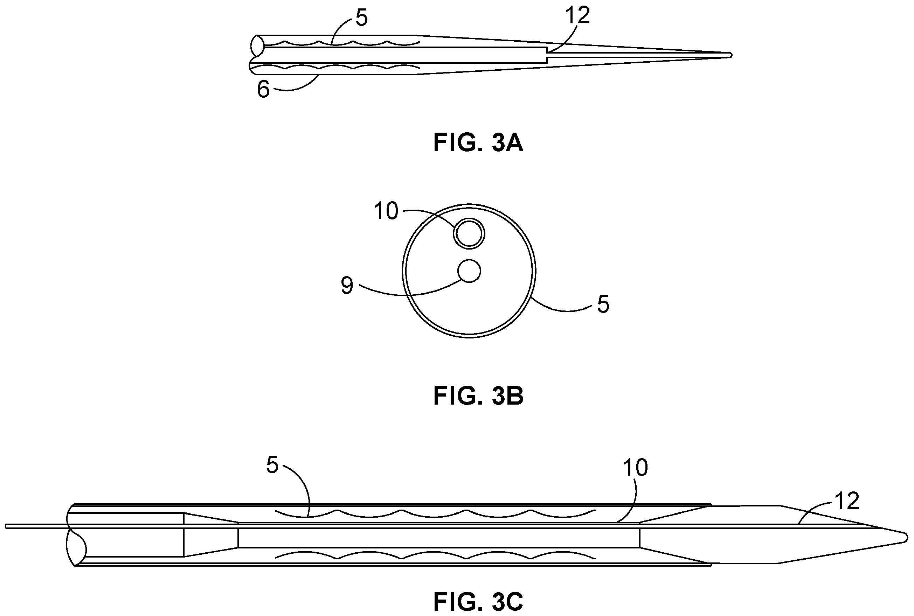

Figures 3a to 3c illustrate a second configuration. The second configuration is similar to the first, but instead of a marker in the tip to define the proximal limit for the imaging probe, the proximal limit is defined by a stepped profile 12 of the designated lumen 10 at a known distance from the device. The stepped profile 12 is hence at the proximal end of the open-ended lumen, where the diameter of the lumen is reduced, and prevents the imaging probe from being advanced any further. It thus provides a known position or reference point relative to the stent device 5. The reduced diameter section of the lumen is however open ended to allow air to be flushed from the lumen.

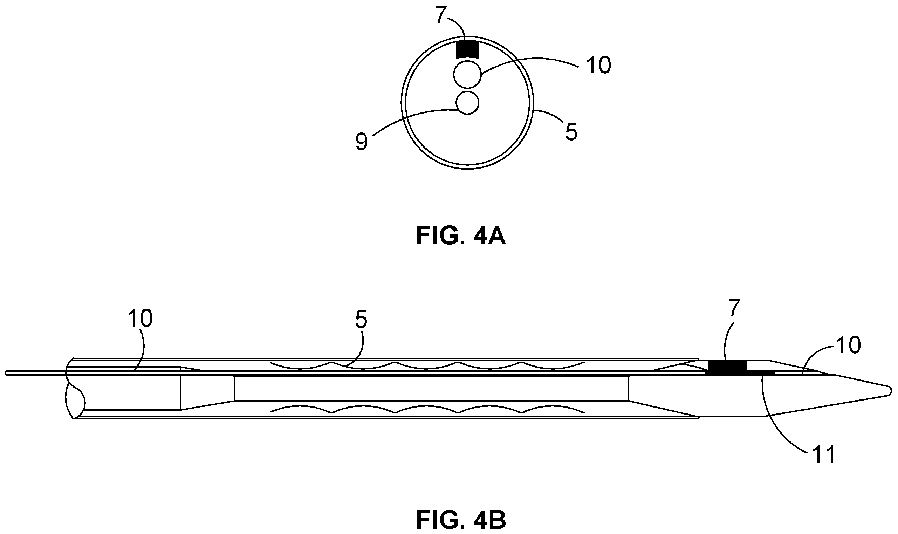

Figures 4a and 4b show a third configuration, which externalizes the imaging probe from the shaft 2 but still inside the compacted device, whereby the probe emerges from a portion of the navigation pathway.

The imaging probe may hence have a designated lumen 10 independent of the shaft lumen 2 in the multi-lumen tube of the delivery apparatus. The visualisation section is primarily a free space between the inside of the compacted device 5 and outside of the shaft 2 over a distance between the exit of the navigation pathway lumen at the distal end of the apparatus and the tip of the shaft which the imaging probe enters to find its proximal limit. The proximal limit may be defined by a marker 7 or a stepped profile in the shaft tip portion 8, as per the prior configuration.

An embodiment is shown in Figures 5a to 5e, which further externalises the imaging probe by a double-sheathed configuration.

In this respect, the imaging probe lumen 10 extends between the exterior of an inner sheath 13 that constrains the compacted device 5 and the interior of an outer sheath 14. A marker 7 is provided in the tip portion 8.

A method of use of the this embodiment is described with reference to Figure 5c to 5e. As shown in Figure 5c, delivery of the medical device involves the vessel being road-mapped using the apparatus. The apparatus is advanced to an appropriate position by detecting environmental cues (e.g. markers on other devices or vascular features).

To deploy the device as shown in Figure 5d, before unsheathing the device, the imaging probe is withdrawn down the visualization pathway distally until the probe detects the distal marker on the device and confirms its position relative to the road-mapped branch vessel above which the device should be deployed.

Figure 5e shows probe interchanging and repositioning.

In this respect, for multi-implant procedures using multiple delivery apparatus, the probe is withdrawn from one compatible delivery apparatus and can be inserted into another compatible delivery apparatus. This is particularly useful if an earlier delivery apparatus must remain in the anatomy in partial deployment while deploying another device, and the first apparatus requires the probe to complete deployment.

To check or adjust apposition, the imaging probe is positioned along the visualization section in the delivery apparatus of the repositionable device, such as aligned with the end stents, and real-time imaging of stent repositioning is enabled.

With this embodiment, there is provided an integrated imaging and medical device delivery apparatus, system and method comprising one or more of the following preferred features:-
a) A self-expanding stent graft medical device
   - with radiopaque markers; and
   - compacted and constrained by an inner sheath.
b) A navigation pathway defined by
   - at the distal end a designated lumen outer of any lumens of the multi-lumen tube carrying support elements of the stent graft and the inner sheath, which opens to allow the imaging probe to emerge on the outside of the inner sheath and its contained medical device;
   - a visualization section that extends from the open end of the designated lumen to a proximal limit of the delivery apparatus;
   - optionally at the proximal end a lumen within a catheter tip; and
   - optionally one or more intermediate guide elements attached externally to the inner sheath or internally to an outer sheath for the imaging probe to be guided from the distal end of the visualization section to the proximal end without deviating from a linear path.
c) An outer sheath encasing the multi-lumen shaft, imaging probe when integrated, and a compacted device constrained by an inner sheath.
d) An imaging probe on an independent catheter
   - of a size compatible with the designated lumen;
   - contained by the outer sheath until the sheath is withdrawn, and external to the inner sheath containing the medical device; and
   - controlled by standard components such as Tuohy Borst valves to be advanced, withdrawn or fixed in position to prevent unwanted displacement of the imaging probe.
e) A spectrally detectable marker attached at the tip defining the proximal limit of the visualization pathway at a known distance from the proximal end of the medical device.

The method of use can also be embodied differently, not only to reflect the different apparatus embodiments, but also the procedural applications.

Another method of use that employs the essential functions of the invention - in whichever physical embodiment - is to reuse the imaging probe already used in a first delivery system (e.g. for a device positioned just below branches) by interchanging and integrating the same probe into another delivery apparatus (e.g. of a device to be positioned just above branch vessels).

The first and second configurations with the navigation pathway within a central shaft enable integration of an imaging probe without altering or increasing the profile size of the delivery apparatus.

The embodiment, having the double sheath and the navigation pathway external to the medical device while still part of the same multi-lumen enclosure, allows imaging of the external environment above and below the device before its deployment, while able to advance internally through the expanded device to inspect the site without interference with the vessel wall.

All embodiments seek to be agnostic to the type of imaging probe, only limited in compatibility of the imaging catheter diameter to the size of the navigation pathway along its length.

All embodiments further seek to provide inter-operability to exchange imaging probes between delivery apparatus during a surgical procedure.

All embodiments additionally seek to provide measurable manoeuvrability of the imaging probe to assess positioning at either end of the medical device relative to the vessel roadmap.

## Claims

1. Integrated imaging and medical device delivery apparatus (1) comprising;
retaining means for retaining a deployable medical device (5); and
an imaging probe lumen (10) defining a navigation pathway (3) through the apparatus (1), the navigation pathway (3) allowing an imaging probe (11) to be reciprocally axially movable through the apparatus (1), the apparatus further comprising one or more reference points along the navigation pathway, providing positional information relative to a retained deployable medical device (5), wherein the imaging probe lumen (10) extends within the apparatus (1) and externally of a deployable medical device (5) when being retained; wherein the apparatus further comprises inner and outer sheaths, the inner sheath (13) for retaining a medical device (5) against deployment and the outer sheath (14) encapsulating the said imaging probe lumen between the inner sheath (13) and the exterior of the apparatus (1).

2. Apparatus (1) according to claim 1, wherein the one or more reference points comprise markers (7) positioned along the length of the navigation pathway (3).

3. Apparatus (1) according to claim 1, wherein the one or more reference points comprise a restriction in the imaging probe lumen (10), dimensioned to impede further progression of an imaging probe (11) along the imaging probe lumen (10).

4. Apparatus (1) according to any preceding claim, wherein the apparatus is configured such that in use a deployable medical device (5) is positionable towards a proximal end of the apparatus (1) with its proximal end spaced from the proximal end of the apparatus (1) by a predetermined amount.

5. Apparatus (1) according to claim 1, wherein there is a second imaging probe lumen passing centrally through the apparatus (1) for allowing an imaging probe (11) to be introduced internally within a deployable medical device (5).

6. Apparatus (1) according to any preceding claim, wherein the deployable medical device (5) comprises a substantially elongate tubular member.

7. Apparatus (1) according to any preceding claim, wherein the deployable medical device (5) comprises a stent device.

8. Apparatus (1) according to any preceding claim, further comprising an atraumatic tip provided at the proximal end of the apparatus (1).

9. Apparatus (1) according to claim 8, wherein the or each imaging probe lumen (10) extends into said tip (8).

10. Apparatus (1) according to claim 3 and 8, wherein the restriction is configured to impede movement of an imaging probe along said tip (8).

11. Apparatus (1) according to any preceding claim, wherein the navigation pathway comprises a visualization section, the reference points being provided within the visualization section for enabling measurement and orientation awareness for the imaging probe.

12. A medical device delivery system, comprising:
an integrated imaging and medical device delivery apparatus (1) according to any one of claims 1 to 11;
an expandable sheathed medical device (5) retained by said retaining means; and,
an imaging probe (11) compatible with said imaging probe lumen (10).

## Patentansprüche

1. Eine integrierte Bildgebungs- und Medizinproduktteinbringungsvorrichtung (1), die Folgendes beinhaltet:
ein Haltemittel zum Halten eines entfaltbaren Medizinprodukts (5); und
ein Bildgebungssondenlumen (10), das einen Navigationspfad (3) durch die Vorrichtung (1) definiert, wobei der Navigationspfad (3) es einer Bildgebungssonde (11) erlaubt, sich axial durch die Vorrichtung (1) hin und her bewegen zu können, wobei die Vorrichtung ferner einen oder mehrere Referenzpunkte entlang dem Navigationspfad beinhaltet, wobei relativ zu einem gehaltenen entfaltbaren Medizinprodukt (5) Positionsinformationen bereitgestellt werden, wobei sich das Bildgebungssondenlumen (10) innerhalb der Vorrichtung (1) und außerhalb eines entfaltbaren Medizinprodukts (5) erstreckt, wenn dieses gehalten wird; wobei die Vorrichtung ferner eine innere und eine äußere Hülle beinhaltet, wobei die innere Hülle (13) dazu dient, ein Medizinprodukt (5) gegen die Entfaltung zu halten und die äußere Hülle (14) das Bildgebungssondenlumen zwischen der inneren Hülle (13) und der Außenseite der Vorrichtung (1) einkapselt.

2. Vorrichtung (1) gemäß Anspruch 1, wobei der eine oder die mehreren Referenzpunkte Markierungen (7) beinhalten, die entlang der Länge des Navigationspfads (3) positioniert sind.

3. Vorrichtung (1) gemäß Anspruch 1, wobei der eine oder die mehreren Referenzpunkte eine Verengung in dem Bildgebungssondenlumen (10) beinhalten, die bemessen ist, um weiteres Fortschreiten einer Bildgebungssonde (11) entlang dem Bildgebungssondenlumen (10) zu behindern.

4. Vorrichtung (1) gemäß einem der vorhergehenden Ansprüche, wobei die Vorrichtung so konfiguriert ist, dass bei Verwendung ein entfaltbares Medizinprodukt (5) in Richtung eines proximalen Endes der Vorrichtung (1) positioniert werden kann, wobei sein proximales Ende von dem proximalen Ende der Vorrichtung (1) um einen vorbestimmten Umfang beabstandet ist.

5. Vorrichtung (1) gemäß Anspruch 1, wobei es ein zweites Bildgebungssondenlumen gibt, das zentral durch die Vorrichtung (1) verläuft, um es einer Bildgebungssonde (11) zu erlauben, intern innerhalb eines entfaltbaren Medizinprodukts (5) eingeführt zu werden.

6. Vorrichtung (1) gemäß einem der vorhergehenden Ansprüche, wobei das entfaltbare Medizinprodukt (5) ein im Wesentlichen längliches röhrenförmiges Element beinhaltet.

7. Vorrichtung (1) gemäß einem der vorhergehenden Ansprüche, wobei das entfaltbare Medizinprodukt (5) eine Stenteinrichtung beinhaltet.

8. Vorrichtung (1) gemäß einem der vorhergehenden Ansprüche, die ferner eine atraumatische Spitze beinhaltet, die an dem proximalen Ende der Vorrichtung (1) bereitgestellt ist.

9. Vorrichtung (1) gemäß Anspruch 8,
wobei sich das oder jedes Bildgebungssondenlumen (10) in die Spitze (8) erstreckt.

10. Vorrichtung (1) gemäß Anspruch 3 und 8, wobei die Verengung konfiguriert ist, um Bewegung einer Bildgebungssonde entlang der Spitze (8) zu behindern.

11. Vorrichtung (1) gemäß einem der vorhergehenden Ansprüche, wobei der Navigationspfad einen Visualisierungsabschnitt beinhaltet, wobei die Referenzpunkte innerhalb des Visualisierungsabschnitts bereitgestellt sind, um Messungs- und Ausrichtungswahrnehmung für die Bildgebungssonde zu ermöglichen.

12. Ein Medizinprodukteinbringungssystem, das Folgendes beinhaltet:
eine integrierte Bildgebungs- und Medizinprodukteinbringungsvorrichtung (1) gemäß einem der Ansprüche 1 bis 11;
ein erweiterbares umhülltes Medizinprodukt (5), das von dem Haltemittel gehalten wird; und
eine Bildgebungssonde (11), die mit dem Bildgebungssondenlumen (10) kompatibel ist.

## Revendications

1. Appareil d'imagerie et de mise en place de dispositif médical intégré (1) comprenant :
un moyen de retenue servant à retenir un dispositif médical déployable (5) ; et
une lumière pour sonde d'imagerie (10) définissant une voie de navigation (3) à travers l'appareil (1), la voie de navigation (3) permettant à une sonde d'imagerie (11) d'être en mesure d'être déplacée axialement en va-et-vient à travers l'appareil (1), l'appareil comprenant en outre un ou plusieurs points de référence le long de la voie de navigation, fournissant des informations de position relatives à un dispositif médical déployable (5) retenu, où la lumière pour sonde d'imagerie (10) s'étend au sein de l'appareil (1) et à l'extérieur d'un dispositif médical déployable (5) lorsqu'il est retenu ;
l'appareil comprenant en outre des gaines interne et externe, la gaine interne (13) servant à retenir un dispositif médical (5) à l'encontre de son déploiement et la gaine externe (14) encapsulant ladite lumière pour sonde d'imagerie entre la gaine interne (13) et l'extérieur de l'appareil (1).

2. Appareil (1) selon la revendication 1, où les un ou plusieurs points de référence comprennent des repères (7) positionnés sur la longueur de la voie de navigation (3).

3. Appareil (1) selon la revendication 1, où les un ou plusieurs points de référence comprennent un resserrement dans la lumière pour sonde d'imagerie (10), dimensionné pour entraver la progression plus avant d'une sonde d'imagerie (11) le long de la lumière pour sonde d'imagerie (10).

4. Appareil (1) selon n'importe quelle revendication précédente, l'appareil étant configuré de telle sorte que, lors de l'utilisation, un dispositif médical déployable (5) est positionnable vers une extrémité proximale de l'appareil (1), son extrémité proximale étant espacée de l'extrémité proximale de l'appareil (1) d'une quantité prédéterminée.

5. Appareil (1) selon la revendication 1, où il y a une deuxième lumière pour sonde d'imagerie qui passe centralement à travers l'appareil (1) servant à permettre à une sonde d'imagerie (11) d'être introduite de manière interne au sein d'un dispositif médical déployable (5).

6. Appareil (1) selon n'importe quelle revendication précédente, où le dispositif médical déployable (5) comprend un élément tubulaire substantiellement allongé.

7. Appareil (1) selon n'importe quelle revendication précédente, où le dispositif médical déployable (5) comprend un dispositif formant endoprothèse.

8. Appareil (1) selon n'importe quelle revendication précédente, comprenant en outre une pointe atraumatique prévue au niveau de l'extrémité proximale de l'appareil (1).

9. Appareil (1) selon la revendication 8,
où la lumière ou bien chaque lumière pour sonde d'imagerie (10) s'étend jusque dans ladite pointe (8).

10. Appareil (1) selon la revendication 3 et la revendication 8, où le resserrement est configuré pour entraver le déplacement d'une sonde d'imagerie le long de ladite pointe (8).

11. Appareil (1) selon n'importe quelle revendication précédente, où la voie de navigation comprend une section de visualisation, les points de référence étant prévus au sein de la section de visualisation pour rendre possible la connaissance de la mesure et de l'orientation pour la sonde d'imagerie.

12. Un système de mise en place de dispositif médical, comprenant :
un appareil d'imagerie et de mise en place de dispositif médical intégré (1) selon l'une quelconque des revendications 1 à 11 ;
un dispositif médical gainé expansible (5) retenu par ledit moyen de retenue ; et,
une sonde d'imagerie (11) compatible avec ladite lumière pour sonde d'imagerie (10).
